# EUROPÄISCHE PATENTSCHRIFT

(11) **EP 3 466 403 B1**
(45) Veröffentlichungstag und Bekanntmachung des Hinweises auf die Patenterteilung: **11.09.2024**
(21) Anmeldenummer: 17194745.0
(22) Anmeldetag: 04.10.2017
(51) Int. Cl.: A61K 8/60, A61K 8/02, A61K 31/7004, A61Q 11/00

(54) **VERWENDUNG EINES MONOSACCHARIDS ZUR SUB- UND/ODER SUPRAGINGIVALEN ZAHNREINIGUNG**
USE OF A MONOSACCHARIDE FOR SUB AND/OR SUPRAGINGIVAL CLEANING OF TEETH
UTILISATION D'UN MONOSACCHARIDE POUR LE NETTOYAGE DE DENTS SUBGINGIVAL ET/OU SUPRAGINGIVAL

(43) Veröffentlichungstag der Anmeldung: 10.04.2019
(73) Patentinhaber: Prisman GmbH, 64653 Lorsch (DE)
(72) Erfinder: METZGER, STEPHAN, 64653 Lorsch (DE); SCHMALZBAUER, BJÖRN, 74635 Neu-Kupfer (DE); BENZING, KATHRIN, 69121 Heidelberg (DE)
(74) Vertreter: Grünecker Patent- und Rechtsanwälte PartG mbB

(56) Entgegenhaltungen:
- WO-A1-2013/191903
- WO-A1-2016/062742
- US-A1- 2003 099 602
- DATABASE WPI Week 201305, Derwent World Patents Index; AN 2013-A28298, XP002776479

## Beschreibung

Die vorliegende Erfindung betrifft nicht-therapeut die Verwendung eines Pulvers fürdie sub- und/oder supragingivalen Pulverstrahlreinigung von ein Pulver zur therapeutischen Verwendung für die sub- und/oder supragingivale Pulverstrahlreinigung von Zahnoberflächen, wobei das Pulver unter Verwendung von zumindest einem Monosaccharid hergestellt wird.

Im Rahmen einer modernen Prophylaxebehandlung ist es heute üblich, die Zahnhartsubstanz von harten und weichen Belegen zu reinigen. Die Pulverstrahlreinigung stellt hierbei ein übliches Verfahren zur professionellen Reinigung von Zahn- bzw. Zahnwurzeloberflächen dar. Verfärbungen und Beläge lassen sich damit in geeigneter Weise entfernen, wodurch ein mechanisches Abkratzen mit Handinstrumenten oder Ultraschallbehandlungen ersetzt bzw. ergänzt werden können.

Zahnoberflächenbehandlungen werden mittels Druckluft und feinen Pulverpartikeln je nach der Abrasionsstärke in Form einer sogenannten Luftabrasion (herkömmliches Pulverstrahlen) oder eines Airpolishing (Luft-Pulver-Wasserstrahlgeräte = LPW) durchgeführt. Beiden Verfahren liegt das Prinzip zugrunde, dass durch das Auftreffen eines Pulver-Druckluft-Gemisches auf die Zahn(wurzel)oberfläche darauf haftende Ablagerungen beseitigt werden. Beim Airpolishing wird dem Luft-Pulver-Gemisch zusätzlich Wasser beigefügt.

Natriumhydrogencarbonat wird seit über 20 Jahren in der Pulverstrahlreinigung als Bestandteil der hierfür verwendeten Pulver eingesetzt. Natriumhydrogencarbonat weist eine gute Verträglichkeit für die zu behandelnden Patienten auf, es kommt jedoch bei der Reinigung von exponierten Wurzel- oder Dentinoberflächen zu einem signifikanten Substanzabtrag.

Als Alternative zu Natriumhydrogencarbonat wurden in der subgingivalen Zahnoberflächenreinigung Zuckeraustauschstoffe und kristalline Aminosäuren als Bestandteil schwach abrasiver Pulver eingesetzt.

So offenbart die EP 2228175 B1 die Verwendung eines Pulvers oder Pulvergemisches zur Herstellung eines Mittels zur Pulverstrahlreinigung von Zahnoberflächen, wobei das Pulver ein Alditol, beispielsweise Erythrit, enthält.

Die Verwendung feinkörniger Pulver und/oder Pulvergemische zur Herstellung eines Mittels für die Pulverstrahlreinigung von supragingivalen Zahnoberflächen wird ferner in der DE 10066408 B4 beschrieben. Die Pulver bzw. Pulvergemische werden hierbei ausgewählt aus Aminosäuren, organischen Säuren und deren Salzen.

Weiterhin wird die Verwendung eines Pulvers mit einer mittleren Korngröße von nicht mehr als 45 µm und einer Dichte von nicht mehr als 2,0 g/cm³ zur Herstellung eines Mittels für die Pulverstrahlreinigung von subgingivaler Zahnhartsubstanz in der EP 1162940 B1 offenbart

Schließlich beschreibt die DE 102014115412 A1 die Verwendung eines Disaccharids als Abrasiv- und/oder Polierkörper zur Herstellung eines dentalen Strahlpulvers für die supra- und/oder subgingivale Pulverstrahlreinigung von Zahnoberflächen.

WO 2013/191903 A1 offenbart eine pulverförmige Zusammensetzung zum Lufipolieren der Oberfläche von hartem Zahngewebe. WO 2016/062742 A1 offenbart ein Pulver für die dentale Pulverstrahlreinigung. US 2003/0099602 A1 offenbart D-Tagatose als Anti-Biofilmmittel. Ein wachstumshemmendes Mittel für Zahnbakterien, das Galaktose und Sorbit enthält, wird offenbart in DATABASE WP1 Week 201305, Thomson Thomson Scientific, London. GB; AN 2013-A28298.

Viele bekannte Pulver weisen jedoch geschmackliche oder physiologische Nachteile auf und rufen bei den Patienten ein unangenehmes Mundgefühl hervor oder wirken abführend.

Aufgabe der vorliegenden Erfindung ist es daher, ein Pulver für die sub- und/oder supragingivale Pulverstrahlreinigung zur Verfügung zu stellen, das eine optimierte abrasive Wirkung und eine verbesserte Patientenakzeptanz aufweist.

Der Gegenstand der Erfindung ist in den beigefögten Ansprüchen beschrieben.

Ein Aspekt der Erfindung bezieht sich auf die nicht-therapeutische Verwendung eines Pulvers für die sub- und/oder supragingivale Pulverstrahlreinigung von Zahnoberflächen, wobei das Pulver unter Verwendung von zumindest einem Monosaccharid hergestellt wird und wobei das Pulver 95 bis 99,9 Gew.% von zumindest einem Monosaccharid und 0,1 bis 5 Gew.% von mindestens einem weiteren Stoff, ausgewählt aus der Gruppe, bestehend aus einer Rieselhilfe, einem Bleichmittel, einem Analgetikum, einem Bakteriozid und/oder einem Geschmacksstoff, enthält.

Ein weiterer Aspekt der Erfindung bezieht sich auf Pulver zur therapeutischen Verwendung für die sub- und/oder supragingivale Pulverstrahlreinigung von Zahnoberflächen, wobei das Pulver unter Verwendung von zumindest einem Monosaccharid hergestellt wird und wobei das Pulver 95 bis 99,9 Gew.% von zumindest einem Monosaccharid und 0,1 bis 5 Gew.% von mindestens einem weiteren Stoff, ausgewählt aus der Gruppe, bestehend aus einer Rieselhilfe, einem Bleichmittel, einem Analgetikum, einem Bakteriozid und/oder einem Geschmacksstoff, enthält.

Ein weiterer Aspekt der Erfindung bezieht sich auf Monosaccharid zur Verwendung in einem dentalen Strahlpulver zur Therapie und/oder Prophylaxe von Zahnwurzel- bzw. Zahnerkrankungen und wobei das Strahlpulver 95 bis 99,9 Gew.% von zumindest einem Monosaccharid und 0,1 bis 5 Gew.% von mindestens einem weiteren Stoff, ausgewählt aus der Gruppe, bestehend aus einer Rieselhilfe, einem Bleichmittel, einem Analgetikum, einem Bakteriozid und/oder einem Geschmacksstoff, enthält,

Bevorzugte Ausführungsformen sind in den abhängigen Ansprüchen beschrieben.

In einer bevorzugten Ausführungsform in Verbindung mit irgendeiner der oben- oder untenstehenden Ausführungsformen ist das Monosaccharid ausgewählt aus der Gruppe, bestehend aus Triosen, Tetrosen, Pentosen und Hexosen, insbesondere aus Pentosen und Hexosen.

In einer bevorzugten Ausführungsform in Verbindung mit irgendeiner der oben- oder untenstehenden Ausführungsformen ist das Monosaccharid ausgewählt aus der Gruppe der Triosen, insbesondere Glycerinaldehyd.

In einer bevorzugten Ausführungsform in Verbindung mit irgendeiner der oben- oder untenstehenden Ausführungsformen ist das Monosaccharid ausgewählt aus der Gruppe der Tetrosen, insbesondere aus Aldotetrosen und Ketotetrosen.

In einer bevorzugten Ausführungsform in Verbindung mit irgendeiner der oben- oder untenstehenden Ausführungsformen ist das Monosaccharid ausgewählt aus der Gruppe der Aldotetrosen, insbesondere aus Erythrose und Threose.

In einer bevorzugten Ausführungsform in Verbindung mit irgendeiner der oben- oder untenstehenden Ausführungsformen ist das Monosaccharid ausgewählt aus der Gruppe der Ketotetrosen, insbesondere Erythrulose.

In einer bevorzugten Ausführungsform in Verbindung mit irgendeiner der oben- oder untenstehenden Ausführungsformen ist das Monosaccharid ausgewählt aus der Gruppe der Pentosen, insbesondere aus Aldopentosen und Ketopentosen,

In einer bevorzugten Ausführungsform in Verbindung mit irgendeiner der oben- oder untenstehenden Ausführungsformen ist das Monosaccharid ausgewählt aus der Gruppe, bestehend aus Aldopentosen" insbesondere aus Ribose, Arabinose. Xylose und Lyxose.

In einer bevorzugten Ausführungsform in Verbindung mit irgendeiner der oben- oder untenstehenden Ausführungsformen ist das Monosaccharid ausgewählt aus der Gruppe, bestehend aus Ketopentosen, insbesondere aus Ribulose und Xylulose.

In einer bevorzugten Ausführungsform in Verbindung mit irgendeiner der oben- oder untenstehenden Ausführungsformen ist das Monosaccharid ausgewählt aus der Gruppe der Hexosen, insbesondere aus Aldohexosen und Ketohexosen.

In einer bevorzugten Ausführungsform in Verbindung mit irgendeiner der oben- oder untenstehenden Ausführungsformen ist das Monosaccharid ausgewählt aus der Gruppe, bestehend aus Aldohexosen, stärker bevorzugt aus Allose, Altrose, Glucose, Mannose, Gulose, Idose, Galactose und Talose, insbesondere Galactose.

In einer bevorzugten Ausführungsform in Verbindung mit irgendeiner der oben- oder untenstehenden Ausführungsformen ist das Monosaccharid ausgewählt aus der Gruppe, bestehend aus Ketohexosen, stärker bevorzugt aus Psicose, Fructose, Sorbose und Tagatose, insbesondere Tagatose.

In einer bevorzugten Ausführungsform in Verbindung mit irgendeiner der oben- oder untenstehenden Ausführungsformen ist das Monosaccharid ausgewählt aus der Gruppe, bestehend aus Galactose und Tagatose. Diese Monosaccharide sind besonders bevorzugt, da Galactose und Tagatose einen niedrigen glykämischen Index aufweisen (Galactose: 20, Tagatose: 3) Weiterhin gelten beide Zucker als "zahnfreundlich", wobei Galactose 20-mal weniger kariogen als Glucose/Fructose und Tagatose nicht kariogen ist. Die Zucker, insbesondere Tagatose, zeigen ferner ein gutes Sprühverhalten.

Alle Monosaccharide können in D- oder L-Form nach Fischer-Projektion verwendet werden.

Das Monosaccharid wirkt in dem Pulver für die sub- und/oder supragingivale Pulverstrahlreinigung von Zahnoberflächen als Abrasiv- und/oder Polierkörper.

In einer bevorzugten Ausführungsform in Verbindung mit irgendeiner der oben- oder untenstehenden Ausführungsformen weist das Pulver eine mittlere Korngröße von < 50 µm, stärker bevorzugt von 10-30 µm, insbesondere von 12-20 µm, auf. Diese mittleren Korngrößen haben sich als besonders vorteilhaft im Hinblick auf die Förderung im Pulverstrahlgerät sowie die abrasiven Eigenschaften gegenüber Zahnfleisch erwiesen. Ein zu feines Pulver kann Probleme bei der Förderung im Pulverstrahlgerät verursachen und ein zu grobes Pulver ist zu abrasiv bzw. aggressiv gegenüber Zahnfleisch.

Die Monosaccharide liegen erfindungsgemäß als Pulver mit einer bestimmten durchschnittlichen Partikelgröße vor. Das Pulver wird dabei durch Mahlen, Sieben, Windsichten, Klassieren und andere dem Fachmann bekannte Prozessschritte in der gewünschten Kornverteilung hergestellt. Für die Abrasivität eines Strahlpulvers sind im Wesentlichen neben der durch das entsprechende Gerät zu beeinflussenden Partikelgeschwindigkeit, Faktoren wie die Härte und Masse der Partikel maßgeblich. Große Partikel mit einer geringeren Dichte haben bei gleicher Masse in etwa die gleiche Abrasivität wie kleinere Teilchen mit größerer Dichte, Ganz wesentlich ist zudem die Korngeometrie, wobei runde Körner weniger abrasiv als scharfkantige Körner sind.

Die mittlere Korngröße, wie hierin verwendet, bezeichnet eine mittlere volumenbezogene Partikelgröße d(50), die nach folgendem Verfahren bestimmt wurde Die volumenbezogene Partikelgröße wurde mittels Laserbeugung mit einem Partikelgrößenanalysegerät (Mastersizer 2000; Lichtquellen: rot: Helium-Neon Laser, 633 nm, blau: LED, 466 nm; Partikelgrößenverteilungsspektrum: 0,02-2000 µm; Softwareversion 5.61, MALVERN Instruments Ltd, Malvern, UK) bestimmt. Als Zelle wurde die Trocken-Dispergiereinheit Scirocco 2000 mit einem Dispergierluftdruck von 1 bar und einer Zuführrate (Rüttelrinne) von 70% (MALVERN Instruments Ltd, Malvern, UK) gewählt. Die Probe wurde direkt nach Herstellung des Monosaccharids entnommen und vermessen. Die Auswertung der Rohdaten erfolgte über die Fraunhofer-Theorie. Es wird hierin die volumenbezogene Partikelgrößenverteilung angegeben. Bei den folgenden angegebenen mittleren Korngrößen handelt es sich um den d(50)-Wert, welcher als mittlere Partikelgröße definiert ist. Unter dem d(50)-Wert wird die Summenhäufigkeitsverteilung der volumengemittelten Größenverteilungsfunktion verstanden. Der d(50)-Wert gibt an, dass 50% der vermessenen Partikel einen Durchmesser aufweisen, der kleiner oder gleich diesem Wert ist. Dies gilt gleichermaßen für den optional zu bestimmenen d(90)- und d(10)-Wert. Hier sind entsprechend 90% bzw. 10% der Partikel kleiner oder gleich dem jeweiligen Wert. Zur Charakterisierung einer Partikelgrößenverteilung wird der Wert d(50) und optional die Werte d(10) und d(90) herangezogen. Wie oben ausgeführt, beschreibt der d(50)-Wert die mittlere volumenbezogene Partikelgröße, die Werte d(10) und d(90) beschreiben die Breite der volumenbezogenen Partikelgrößenverteilung.

In einer bevorzugten Ausführungsform in Verbindung mit irgendeiner der oben- oder untenstehenden Ausführungsformen enthält das Mittel für die Pulverstrahlreinigung von sub- und/oder supragingivaler Zahnhartsubstanz mindestens einen weiteren Stoff, ausgewählt aus der Gruppe, bestehend aus einer Rieselhilfe, einem Bleichmittel, einem Analgetikum, einem Bakteriozid, einem Alditol, einer kristallinen Aminosäure und/oder einem Geschmacksstoff.

In einer bevorzugten Ausführungsform in Verbindung mit irgendeiner der oben- oder untenstehenden Ausführungsformen ist die Rieselhilfe ausgewählt aus der Gruppe, bestehend aus Siliziumdioxid, Calciumcarbonat, Aluminiumsilikat und/oder Aluminiumhydroxid, stärker bevorzugt Siliziumdioxid, insbesondere pyrogenes Siliziumdioxid (pyrogene Kieselsäure), Rieselhilfen stellen Trennmittel dar, die kristallinen Substanzen zugesetzt werden, um das Zusammenklumpen der Substanzen zu vermeiden und somit einen besseren maschinellen Einsatz zu ermöglichen. Der Einsatz einer oder mehrere Rieselhilfen kann auch bei Strahlpulvern erforderlich sein, um eine Verstopfung der Düsen des Strahlgeräts bei einer Verklumpung der Pulversubstanzen zu vermeiden.

In einer bevorzugten Ausführungsform in Verbindung mit irgendeiner der oben- oder untenstehenden Ausführungsformen ist das Bleichmittel ausgewählt aus der Gruppe, bestehend aus Peroxydverbindungen, Kalium-, Ammonium-, Natrium- und Lithiumpersulfaten und Perboratmono- und -tetrahydraten, Natriumpyrophosphatperoxyhydrat und Magnesium-, Calcium-, Strontium- und Zinkperoxiden.

In einer bevorzugten Ausführungsform in Verbindung mit irgendeiner der oben- oder untenstehenden Ausführungsformen ist das Analgetikum ausgewählt aus Articain oder Lidocain.

In einer bevorzugten Ausführungsform in Verbindung mit irgendeiner der oben- oder untenstehenden Ausführungsformen ist das Bakteriozid ausgewählt aus der Gruppe, bestehend aus Chlorhexidin, Triclosan, Kupfer-, Zink- und Zinn(II)salzen, wie Zinkcitrat, Zinksulfat, Zinkglycinat, Natriumzinkcitrat und Zinn(II)pyrophosphat, Metronidazol, quaternären Ammoniumverbindungen, Bisguaniden, wie Chlorhexidindigluconat, Hexetidin, Cetylpyridiniumchlorid, Octenidin und Alexidin.

In einer bevorzugten Ausführungsform in Verbindung mit irgendeiner der oben- oder untenstehenden Ausführungsformen ist das Alditol ausgewählt aus der Gruppe, bestehend aus Erythrit, Sorbit, Xylit, Mannit, Isomalt, Lactit, Threit und Arabit. Alditole sind nichtcyclische Polyole und können als Zuckeraustauschstoffe zur Verbesserung des Geschmacks eingesetzt werden. Alditole sind nicht kariogen.

In einer bevorzugten Ausführungsform in Verbindung mit irgendeiner der oben- oder untenstehenden Ausführungsformen ist die kristalline Aminosäure Glycin.

In einer bevorzugten Ausführungsform in Verbindung mit irgendeiner der oben- oder untenstehenden Ausführungsformen ist der Geschmacksstoff ausgewählt aus der Gruppe, bestehend aus natürlichen oder naturidentischen Geruchs- und/oder Geschmackstoffen. Hierunter werden allgemein Aromen verstanden, mit welchen Produkten, die oral eingenommen oder verwendet werden, ein besonderer Geruch oder Geschmack verliehen wird. "Natürliche" aromatisierende Geruchs- und Geschmacksstoffe sind Substanzen oder Substanzgemische, die aus natürlichen Quellen, beispielsweise Pflanzen oder Pflanzenteilen, gewonnen und gegebenenfalls aufgereinigt werden. Alternativ können die einzusetzenden Geschmacksstoffe auch synthetisch hergestellt worden sein.

Die oben genannten weiteren Stoffe müssen im Hinblick auf ihre Partikelgröße so ausgewählt werden, dass die mittlere Korngröße des Pulvers für die sub- und/oder supragingivale Pulverstrahlreinigung von Zahnoberflächen von < 50 µm, von 10-30 µm bzw. von 12-20 µm erhalten bleibt

Das erfindungsgeinäß verwendete Pulver enthält (i) 95-99,5 Gew.% von zumindest einem Monosaccharid und (ii) 0,5-5 Gew.%, von mindestens einem weiteren Stoff, ausgewählt aus der Gruppe, bestehend aus einer Rieselhilfe, einem Bleichmittel, einem Analgetikum, einem Bakteriozid und/oder einem Geschmacksstoff.

Für das Monosaccharid zur Verwendung in einem dentalen Strahlpulver zur Therapie und/oder Prophylaxe von Zahnwurzel- bzw. Zahnerkrankungen gelten die gleichen bevorzugten Ausführungsformen wie für die erfindungsgemäße nicht-therapeutische Verwendung und das erfmdungsgemäße Puh er zur therapeutischen Verwendung beschrieben.

Unter einer subgingivalen Verwendung, wie hierin verwendet, wird der Einsatz eines Strahlpulvers bei Zahnoberflächen verstanden, die unterhalb des Zahnfleischsaumes liegen. Bei einer bedingt durch die Anhäufung von subgingivalen Biofilmen fortschreitenden Entzündungsreaktion kommt es zur Ausbildung von Zahnfleischtaschen, die unter Verwendung von Zahnbürste oder Zahnseide praktisch nicht gereinigt werden können. Da subgingivale Zahnoberflächen weicher und empfindlicher sind als etwa supragingival gelegene Zahnoberflächen, muss die Abrasivität des eingesetzten Strahlpulvers möglichst gering gehalten werden.

Unter einer supragingivaten Verwendung, wie hierin verwendet, wird der Einsatz eines Strahlpulvers bei Zahnoberflächen verstanden, die oberhalb des Zahnfleischsaumes liegen.

Wenn sich Zahnfleisch entzündet und über längere Zeit nicht behandelt wird, kann es zu einer Entzündung des Zahnhalteapparats, d.h. einer Parodontitis, kommen. Diese geht regelmäßig mit einem Knochenabbau und einem Zahnfleischrückgang einher. Infolge des Zahnfleischrückgangs werden die Zahnhälse freigelegt und es kommt zu einer erhöhten Sensibilität. Diese freigelegten Zahnhälse müssen, ebenso wie die üblicherweise subgingival gelegenen Bereiche, insbesondere die Zahnwurzeln, mit Pulver geringer Abrasivität behandelt werden, um das hier vorliegende empfindliche Zahngewebe nicht zu verletzen.

Eine regelmäßige Reinigung der Zahnoberflächen ist essenziell für eine gesunde Mundflora. In jedem Mund bildet sich innerhalb weniger Tage ein Belag auf den Zähnen, Plaque oder auch Biofilm genannt, der aus Milliarden von Bakterien besteht, deren Stoffwechselprodukte die Zähne und das Zahnfleisch schädigen können. Ähnlich wie für die Entstehung von Karies sind Beläge an der Zahnoberfläche, in den Zahnzwischenräumen und vor allem in den Zahnfleischtaschen eine wesentliche Ursache für Entzündungen des Zahnfleischsaums und des Zahnhalteapparats (Parodontopathien).

Ein wichtiges Ziel aller zahnerhaltenden prophylaktischen Maßnahmen zur Karies- und Parodontitisvorbeugung ist daher die Verhinderung der Entstehung von Belägen bzw. die Entfernung von Belägen durch regelmäßige und gründliche Zahnpflege,

Die erfindungsgemäße nicht-therapeutische Verwendung, das erfindungsgemäße Pulver zur therapeutischen Verwendung sowie das erfindungsgemäße Monosaccharid zur Verwendung in einem dentalen Strahlpulver ermöglichen eine erfolgreiche Therapie und/oder Prophylaxe von Zahnwurzel- bzw. Zahnerkrankungen. Hierbei werden durch die erfindungsgemäße nicht-therapeutische Verwendung, das erfindungsgemäße Pulver zur therapeutischen Verwendung sowie das erfindungsgemäße Monosaccharid zur Verwendung in einem dentalen Strahlpulver eine hervorragende Reinigungs- und Politurleistung bei sehr geringer Abrasivität und hervorragender Patientenakzeptanz erzielt.

Die Erfindung wird im Folgenden durch Beispiele erläutert

### Beispiel 1

Es wurden 100 g des Pulvers durch Vermischen von 99,05 g einer Tagatose mit einer mittleren Korngröße d(50) von 15,97 µm (d(10) = 2,39 µm, d(90) = 49,04 µm) 0,45 g pyrogener Kieselsäure und 0,50 g Pfefferminz-Aroma hergestellt.

### Beispiel 2

Es wurden 100 g eines Pulvers durch Vermischen von 99,05 g Tagatose mit einer mittleren Korngröße d(50) von 10 µm 0,45 g pyrogener Kieselsäure und 0,50 g Pfefferminz-Aroma hergestellt.

### Beispiel 3

Es wurden 100 g eines Pulvers durch Vermischen von 98,80 g Tagatose mit einer mittleren Korngröße d(50) von 20 µm, 0,20 g pyrogener Kieselsäure und 1,00 g Kirsch-Aroma hergestellt

### Beispiel 4

Es wurden 100 g eines Pulvers durch Vermischen von 98,70 g Tagatose mit einer mittleren Korngröße d(50) von 25 µm, 0,30 g pyrogener Kieselsäure und 1,00 g Zitronen-Aroma hergestellt.

### Beispiel 5

Es wurden 100 g eines Pulvers durch Vermischen von 98,00 g Tagatose mit einer mittleren Korngröße d(50) von 30 µm. 1.00 g pyrogener Kieselsäure und 1,00 g Zitronen-Aroma hergestellt

### Beispiel 6

Das Tagatosepulver gemäß Beispiel 1 wurde mit verschiedenen bekannten Pulvern auf Natriumhydrogencarbonat-, Glycin-, Erythritol- sowie Trehalosebasis verglichen. Pulver auf Natriumhydrogencarbonatbasis mit einer mittleren Korngröße von 40 µm sind in der Reinigung von Zahnschmelz am weitesten verbreitet. Natriumhydrogencarbonatpulver eignen sich aufgrund ihrer hohen Abrasivität jedoch nicht zur Reinigung von empfindlichen Zahnoberflächen, Zahnhälsen oder im subgingivalen Bereich. Das Tagatosepulver gemäß Beispiel 1 mit einer mittleren Korngröße von 15 µm ist weniger abrasiv als herkömmliche Pulver und weist eine hervorragende Reinigungs- und Politurwirkung auf, wie im Folgenden gezeigt wird.

Die Abrasivität des Tagatosepulvers gemäß Beispiel 1 wurde mit im Handel erhältlichen Pulvern (Air-Flow Classic Comfort, Air-Flow Soft, Air-Flow Plus, Air-Flow Perio von der Firma EMS Electro Medical Systems SA, Schweiz; Lunos Gentle Clean, Lunos Perio Combi von der Firma Dürr Dental AGIOrochemie GmbH + Co.KG, Deutschland) verglichen, wie Tabelle 1 zu entnehmen ist. Die Durchführung der Versuche erfolgte mit einem handelsüblichen Pulverstrahlgerät (Airflow handy 2+, EMS Electro Medical Systems SA, Schweiz). Zur Simulation der Zahnoberfläche kam das Polyetheretherketon PEEK, ein in der dentalen Prothetik eingesetztes Hochleistungspolymer, zum Einsatz. Die Düse des Pulverstrahlgeräts wurde im Abstand von ca. 2 mm über der Polymeroberfläche arretiert und der Pulverstrahl 30 Sekunden bei einem Strahldruck von 4,0 bar aktiviert. Die Tiefe des durch das eingesetzte Pulver abgeschliffenen Bereichs der Polymeroberfläche diente als Maß für dessen Abrasivität, Um statistische Schwankungen zu berücksichtigen, wurde jeder Versuch fünfmal wiederholt. Der Wert von 10 entspricht der Abrasivität von Natriumhydrogencarbonat bei einer mittleren Korngröße von 40 µm (siehe Tabelle 1). Ein niedrigerer Zahlenwert entspricht einer geringeren Abrasivität.

**Tabelle 1: Abrasivität verschiedener Pulver**

| Mittlere Korngröße | ∅ 65 µm | ∅ 40 µm | ∅ 30 µm | ∅ 25 µm | ∅ 16 µm |
|---|---|---|---|---|---|
| Natriumhydrogencarbonat | | 10 | | | |
| Glycin | 9 | | | 6 | |
| Trehalose | 4 | | 2 | | |
| Erythritol | | | | | 3 |
| Tagatose | | | | | 1 |

Weiterhin wurde die Reinigungs- und Politurwirkung des erfindungsgemäß verwendeten Tagatosepulvers gemäß Beispiel 1 im Vergleich zu handelsüblichen Pulvern getestet. Hierfür wurde die Abtragung von Zahnbelägen simuliert. Die Durchführung der Versuche erfolgte mit einem handelsüblichen Pulverstrahlgerät (Airflow handy 2+, EMS Electro Medical Systems SA, Schweiz). Das erfindungsmäße Tagatosepulver sowie die zu vergleichenden Pulver wurden auf eine beschichtete PEEK Platte gestrahlt. Hierbei wurden 1 cm x 1cm große Areale mit den jeweiligen Pulvern so lange behandelt, bis die Beschichtung vollständig von der Fläche entfernt war. Die dafür benötigte Zeit für die Reinigung der Fläche wurde gemessen. Um statistische Schwankungen zu berücksichtigen, wurde jeder Versuch zehnmal wiederholt Zur besseren Vergleichbarkeit der Reinigungswirkung und des Politureffektes wurde dem Natriumhydrogencarbonatpulver mit einen Teilchendurchmesser von 40 µm, wie im Versuch zuvor, der Wert 10 zugeordnet. Die ermittelten Zeiten wurden auf den Wert 10 normiert. Je höher der Punktwert ist, umso besser ist die Reinigungsleistung (siehe Tabelle 2). Es zeigte sich eine besonders schonende Reinigungs- und Politurwirkung des erfindungsgemäß verwendeten Tagatosepulvers gemäß Beispiel 1.

**Tabelle 2: Reinigungswirkung verschiedener Pulver**

| Mittlere Korngröße | ∅ 65 µm | ∅ 40 µm | ∅ 30 µm | ∅ 25 µm | ∅ 16 µm |
|---|---|---|---|---|---|
| Natriumhydrogencarbonat | | 10 | | | |
| Glycin | 13 | | | 15 | |
| Trehalose | 14 | | 13 | | |
| Erythritol | | | | | 14 |
| Tagatose | | | | | 17 |

Zur Beurteilung der Politurwirkung wurde zusätzlich die Optik und Haptik der Oberfläche nach der Abstrahlung des beschichteten Areals bewertet. Auch hier wurde zur besseren Vergleichbarkeit dem Natriumhydrogencarbonatpulver mit einem Teilchendurchmesser von 40 µm der Wert 10 zugeordnet. Je kleiner der Punktwert ist, umso glatter und gleichmäßiger ist die gereinigte Oberfläche (siehe Tabelle 3).

**Tabelle 3: Politurwirkung verschiedener Pulver**

| Mittlere Korngröße | ∅ 65 µm | ∅ 40 µm | ∅ 30 µm | ∅ 25 µm | ∅ 16 µm |
|---|---|---|---|---|---|
| Natriumhydrogencarbonat | | 10 | | | |
| Glycin | 8 | | | 2 | |
| Trehalose | 9 | | 5 | | |
| Erythritol | | | | | 3 |
| Tagatose | | | | | 2 |

Das erfindungsgemäß verwendete Tagatosepulver gemäß Beispiel 1 zeigte eine hervorragende Reinigungs- und Politurleistung bei sehr geringer Abrasivität. Das Glycinpulver mit einem

Teilchendurchmesser von 25 µm sowie das Erythritolpulver mit einem Teilchendurchmesser von 15 µm zeigten ein ähnliches Verhalten bei der Politur der Fläche, allerdings schnitten diese bei der Bewertung der Reinigungszeit und der Abrasivität im Vergleich zu dem erfindungsgemäß verwendeten Tagatosepulver schlechter ab. Dieser Politureffekt macht eine abschließende Politur des Zahns nach der Bestrahlung überflüssig.

Tagatose weist einen niedrigen Glykämischen Index (GI) auf und ist für Diabetiker geeignet. Tagatose ist des Weiteren sehr zahnfreundlich und nicht kariogen. Es zeigt eine hohe Verträglichkeit und wirkt nicht abführend. Tagatose ist weiterhin nicht hygroskopisch und zeigt ein exzellentes Sprühverhalten, es kommt zu keinem Zusammenbacken des Pulvers im Strahlgerät.

## Patentansprüche

1. Nicht-therapeutische Verwendung eines Pulvers für die sub- und/oder supragingivale Pulverstrahlreinigung von Zahnoberflächen, wobei das Pulver unter Verwendung von zumindest einem Monosaccharid hergestellt wird und wobei das Pulver 95 bis 99,9 Gew.% von zumindest einem Monosaccharid und 0,1 bis 5 Gew.% von mindestens einem weiteren Stoff, ausgewählt aus der Gruppe, bestehend aus einer Rieselhilfe, einem Bleichmittel, einem Analgetikum, einem Bakteriozid und/oder einem Geschmacksstoff, enthält.

2. Pulver zur therapeutischen Verwendung für die sub- und/oder supragingivale Pulverstrahlreinigung von Zahnoberflächen, wobei das Pulver unter Verwendung von zumindest einem Monosaccharid hergestellt wird und wobei das Pulver 95 bis 99,9 Gew.% von zumindest einem Monosaccharid und 0,1 bis 5 Gew.% von mindestens einem weiteren Stoff, ausgewählt aus der Gruppe, bestehend aus einer Rieselhilfe, einem Bleichmittel, einem Analgetikum, einem Bakteriozid und/oder einem Geschmacksstoff, enthält.

3. Nicht-therapeutische Verwendung nach Anspruch 1 oder Pulver zur therapeutischen Verwendung nach Anspruch 2, wobei das Monosaccharid ausgewählt ist aus der Gruppe, bestehend aus Pentosen und Hexosen.

4. Nicht-therapeutische Verwendung nach einem der Ansprüche 1 oder 3, oder Pulver zur therapeutischen Verwendung nach einem der Ansprüche 2 oder 3, wobei das Monosaccharid ausgewählt ist aus der Gruppe, bestehend aus Ketohexosen.

5. Nicht-therapeutische Verwendung nach einem der Ansprüche 1, 3 oder 4, oder Pulver zur therapeutischen Verwendung nach einem der Ansprüche 2 bis 4, wobei das Monosaccharid Tagatose ist.

6. Nicht-therapeutische Verwendung nach einem der Ansprüche 1 oder 3, oder Pulver zur therapeutischen Verwendung nach einem der Ansprüche 2 oder 3, wobei das Monosaccharid ausgewählt ist aus der Gruppe, bestehend aus Aldohexosen.

7. Nicht-therapeutische Verwendung nach einem der Ansprüche 1, 3 oder 6, oder Pulver zur therapeutischen Verwendung nach einem der Ansprüche 2, 3 oder 6, wobei das Monosaccharid Galactose ist.

8. Nicht-therapeutische Verwendung nach einem der Ansprüche 1 oder 3 bis 7, oder Pulver zur therapeutischen Verwendung nach einem der Ansprüche 2 bis 7, wobei das Pulver eine mittlere Korngröße von < 50 µm aufweist.

9. Nicht-therapeutische Verwendung nach Anspruch 8 oder Pulver zur therapeutischen Verwendung nach Anspruch 8, wobei das Pulver eine mittlere Korngröße von 10-30 µm aufweist.

10. Nicht-therapeutische Verwendung nach einem der Ansprüche 1 oder 3 bis 9, oder Pulver zur therapeutischen Verwendung nach einem der Ansprüche 2 bis 9, wobei das Mittel für die Pulverstrahlreinigung von sub- und/oder supragingivaler Zahnhartsubstanz mindestens einen weiteren Stoff, ausgewählt aus der Gruppe, bestehend aus einer Rieselhilfe, einem Bleichmittel, einem Analgetikum, einem Bakteriozid, einem Alditol, einer kristallinen Aminosäure und/oder einem Geschmacksstoff enthält.

11. Monosaccharid zur Verwendung in einem dentalen Strahlpulver zur Therapie und/oder Prophylaxe von Zahnwurzel- bzw. Zahnerkrankungen und wobei das Strahlpulver 95 bis 99,9 Gew.% von zumindest einem Monosaccharid und 0,1 bis 5 Gew.% von mindestens einem weiteren Stoff, ausgewählt aus der Gruppe, bestehend aus einer Rieselhilfe, einem Bleichmittel, einem Analgetikum, einem Bakteriozid und/oder einem Geschmacksstoff, enthält.

## Claims

1. Non-therapeutic use of a powder for sub- and/or supragingival powder jet cleaning of tooth surfaces, wherein the powder is prepared using at least one monosaccharide and wherein the powder contains 95 to 99.9% by weight of at least one monosaccharide and 0.1 to 5% by weight of at least one further substance selected from the group consisting of a flow aid, a bleaching agent, an analgesic, a bacteriocide and/or a flavoring.

2. Powder for therapeutic use for sub- and/or supragingival powder jet cleaning of tooth surfaces, wherein the powder is prepared using at least one monosaccharide and wherein the powder contains 95 to 99.9% by weight of at least one monosaccharide and 0.1 to 5% by weight of at least one further substance selected from the group consisting of a flow aid, a bleaching agent, an analgesic, a bacteriocide and/or a flavoring.

3. The non-therapeutic use according to claim 1 or the powder for therapeutic use according to claim 2, wherein the monosaccharide is selected from the group consisting of pentoses and hexoses.

4. The non-therapeutic use according to any one of claims 1 or 3, or the powder for therapeutic use according to any one of claims 2 or 3, wherein the monosaccharide is selected from the group consisting of ketohexoses.

5. Non-therapeutic use according to any one of claims 1, 3 or 4, or powder for therapeutic use according to any one of claims 2 to 4, wherein the monosaccharide is tagatose.

6. The non-therapeutic use according to any one of claims 1 or 3, or the powder for therapeutic use according to any one of claims 2 or 3, wherein the monosaccharide is selected from the group consisting of aldohexoses.

7. Non-therapeutic use according to any one of claims 1, 3 or 6, or powder for therapeutic use according to any one of claims 2, 3 or 6, wherein the monosaccharide is galactose.

8. Non-therapeutic use according to any one of claims 1 or 3 to 7, or powder for therapeutic use according to any one of claims 2 to 7, wherein the powder has an average grain size of < 50 µm.

9. The non-therapeutic use according to claim 8 or the powder for therapeutic use according to claim 8, wherein the powder has an average grain size of 10-30 µm.

10. Non-therapeutic use according to any one of claims 1 or 3 to 9, or powder for therapeutic use according to any one of claims 2 to 9, wherein the agent for powder jet cleaning of sub- and/or supragingival dental hard tissue comprises at least one further substance selected from the group consisting of an anti-caking agent, a bleaching agent, an analgesic, a bacteriocide, an alditol, a crystalline amino acid and/or a flavoring.

11. Monosaccharide for use in a dental jet powder for the therapy and/or prophylaxis of tooth-root or tooth diseases and wherein the jet powder contains 95 to 99.9% by weight of at least one monosaccharide and 0.1 to 5% by weight of at least one further substance selected from the group consisting of an anti-caking agent, a bleaching agent, an analgesic, a bacteriocide and/or a flavoring.

## Revendications

1. Utilisation non thérapeutique d'une poudre pour le nettoyage par jet de poudre sous- et/ou supra-gingival de surfaces dentaires, dans laquelle la poudre est préparée en utilisant au moins un monosaccharide et dans laquelle la poudre contient 95 à 99,9 % en poids d'au moins un monosaccharide et 0,1 à 5 % en poids d'au moins une autre substance choisie dans le groupe constitué par un agent d'écoulement, un agent de blanchiment, un analgésique, un bactéricide et/ou un agent aromatisant.

2. Poudre à usage thérapeutique pour le nettoyage par jet de poudre sous- et/ou supra-gingival de surfaces dentaires, dans laquelle la poudre est préparée en utilisant au moins un monosaccharide et dans laquelle la poudre contient 95 à 99,9 % en poids d'au moins un monosaccharide et 0,1 à 5 % en poids d'au moins une autre substance choisie dans le groupe constitué par un agent d'écoulement, un agent de blanchiment, un analgésique, un bactéricide et/ou un agent aromatisant.

3. Utilisation non thérapeutique selon la revendication 1 ou poudre pour utilisation thérapeutique selon la revendication 2, dans laquelle le monosaccharide est choisi dans le groupe constitué par les pentoses et les hexoses.

4. Utilisation non thérapeutique selon l'une des revendications 1 ou 3, ou poudre pour utilisation thérapeutique selon l'une des revendications 2 ou 3, dans laquelle le monosaccharide est choisi dans le groupe constitué par les cétohexoses.

5. Utilisation non thérapeutique selon l'une quelconque des revendications 1, 3 ou 4, ou poudre pour utilisation thérapeutique selon l'une quelconque des revendications 2 à 4, dans laquelle le monosaccharide est le tagatose.

6. Utilisation non thérapeutique selon l'une des revendications 1 ou 3, ou poudre pour utilisation thérapeutique selon l'une des revendications 2 ou 3, dans laquelle le monosaccharide est choisi dans le groupe constitué par les aldohexoses.

7. Utilisation non thérapeutique selon l'une quelconque des revendications 1, 3 ou 6, ou poudre pour utilisation thérapeutique selon l'une quelconque des revendications 2, 3 ou 6, dans laquelle le monosaccharide est le galactose.

8. Utilisation non thérapeutique selon l'une des revendications 1 ou 3 à 7, ou poudre pour utilisation thérapeutique selon l'une des revendications 2 à 7, ladite poudre ayant une granulométrie moyenne < 50 µm.

9. Utilisation non thérapeutique selon la revendication 8 ou poudre pour utilisation thérapeutique selon la revendication 8, dans laquelle la poudre a une granulométrie moyenne de 10 à 30 µm.

10. Utilisation non thérapeutique selon l'une des revendications 1 ou 3 à 9, ou poudre pour utilisation thérapeutique selon l'une des revendications 2 à 9, dans laquelle l'agent pour le nettoyage par jet de poudre des tissus dentaires durs sous-et/ou supra-gingivaux contient au moins une autre substance choisie dans le groupe constitué par un agent d'aide à l'écoulement, un agent de blanchiment, un analgésique, un bactériocide, un alditol, un acide aminé cristallin et/ou un agent aromatisant.

11. Monosaccharide pour utilisation dans une poudre de sablage dentaire pour la thérapie et/ou la prophylaxie de maladies des racines dentaires ou des dents, et dans lequel la poudre de sablage contient 95 à 99,9 % en poids d'au moins un monosaccharide et 0,1 à 5 % en poids d'au moins une autre substance choisie dans le groupe constitué par un agent d'écoulement, un agent de blanchiment, un analgésique, un bactéricide et/ou un agent aromatisant.
